# FASCICULE DE BREVET EUROPEEN

(11) **EP 2 282 842 B1**
(45) Date de publication et mention de la délivrance du brevet: **12.06.2013**
(21) Numéro de dépôt: 09742297.6
(22) Date de dépôt: 10.04.2009
(51) Int. Cl.: B05B 11/04, A45D 34/02, A61L 9/12, B65D 77/06, B65D 83/00

(54) **DISTRIBUTEUR DE FRAGRANCE**
DUFTSPENDER
FRAGRANCE DISPENSER

(30) Priorité: 25.04.2008 FR 0852806
(43) Date de publication de la demande: 16.02.2011
(73) Titulaire: Aptar France SAS, 27110 Le Neubourg (FR)
(72) Inventeur: LIGNY, Jean-Jacques, 78230 LE PECQ (FR); PENNANEAC'H, Hervé, F-56600 Lanester (FR)
(74) Mandataire: CAPRI
(86) Numéro de dépôt international: PCT/FR2009/050646
(87) Numéro de publication internationale: WO 2009/136099

(56) Documents cités:
- WO-A-92/14607
- FR-A- 2 791 645
- FR-A- 2 852 930
- US-A- 4 817 860
- US-A- 5 188 236
- US-A1- 2003 094 466
- US-A1- 2003 218 024
- US-A1- 2004 000 596

## Description

La présente invention concerne un distributeur de fragrance sous forme vaporisée, et plus particulièrement sous la forme d'un jet d'air chargé de fragrance. Le domaine d'application privilégié de la présente invention est celui de la parfumerie, mais d'autres domaines peuvent également être concernés.

Il existe déjà depuis longtemps des distributeurs de fragrance sous la forme de fines gouttelettes de produit fluide de fragrance. On parle alors de « vaporisateur », comprenant un réservoir de produit fluide sur lequel est monté une pompe actionnable à l'aide d'un ou de plusieurs doigts. L'actionnement de la pompe a pour effet de prélever une dose de produit fluide à l'intérieur du réservoir, de mettre cette dose sous pression, et de la distribuer sous forme de fines gouttelettes divisées. Il est également connu de distribuer le produit fluide en mélange avec de l'air. L'air est mis sous pression pour former un flux qui transporte les fines gouttelettes de fragrance.

D'autre part, on connaît déjà dans l'art antérieur des échantillons, de fragrance par exemple sous la forme de bandes de fragrance, dans lesquels la fragrance est contenue dans des microcapsules que l'on va briser pour libérer la fragrance qu'elles contiennent. De manière conventionnelle, les microcapsules de fragrance se présentent sous la forme d'une couche appliquée sur un substrat telle qu'une feuille. Une autre feuille est disposée sur la couche de microcapsules de fragrance, de sorte que la couche est prise en sandwich entre les deux feuilles. L'utilisateur va tirer sur une des deux feuilles pour la détacher de la couche de microcapsules, ce qui a pour effet de briser les microcapsules qui libèrent alors leur fragrance. Ce type d'échantillon de fragrance est particulièrement bien adapté pour être inclus entre les pages de magazines. La couche de microcapsules de fragrance peut par exemple être disposée à l'intérieur d'un rabat formé par une feuille du magazine. L'utilisateur soulève alors ce rabat qui déchire les microcapsules. La fragrance se libère dans l'air sous forme de gaz ou de vapeur. L'utilisateur peut humer ou sentir la fragrance, d'autant plus qu'il se rapproche de la couche de microcapsules brisées.

Les vaporisateurs à pompe sont particulièrement bien adaptés pour distribuer des fragrances sous forme de gouttelettes liquides. Ils ne peuvent cependant pas être insérés dans un magazine, du fait de l'épaisseur du vaporisateur. De plus, le coût de tels vaporisateurs est relativement élevé, particulièrement pour constituer un échantillon gratuit. D'autre part, les bandes de fragrance utilisant des microcapsules ne permettent pas une distribution ciblée de la fragrance. En effet, la fragrance se libère de manière anarchique en fonction des courants de convection de l'air. L'utilisateur est pratiquement obligé de coller son nez sur la couche de microcapsules brisées où la senteur de la fragrance est mélangée avec les odeurs d'imprimerie et de colle. Ainsi, ni les vaporisateurs de fines gouttelettes, ni les bandes de fragrance ne constituent un distributeur de fragrance approprié à une utilisation en tant qu'échantillon, particulièrement insérable entre les pages d'un magazine.

Dans l'art antérieur, on connaît le document FR2852930 qui décrit un distributeur de jets d'air chargé de fragrance comprenant une poche souple déformable de volume interne variable renfermant un produit fluide, la poche ayant initialement un volume minimal, l'air de la poche étant chargé avec de la fragrance lors de la première entrée d'air dans la poche, la poche comprenant un orifice à travers lequel l'air pénètre dans la poche et sort de la poche en étant chargé avec de la fragrance, lorsque la poche varie de volume.

On connaît aussi le document US5188236 qui décrit un échantillon de fragrance insérable entre les pages d'un magazine sous forme de bande de fragrance dans lequel la fragrance est contenue dans les microcapsules que l'utilisateur va briser lors de la première utilisation pour libérer la fragrance qu'elle contienne.

La présente invention a pour but de remédier aux inconvénients précités des distributeurs de fragrance de l'art antérieur en définissant un nouveau distributeur alliant les avantages à la fois du vaporisateur traditionnel et de la bande de fragrance, sans en reproduire les inconvénients. Le distributeur de l'invention devra être particulièrement plat ou mince de manière à pouvoir être inséré entre les pages d'un magazine. D'autre part, sa fabrication doit être simple et son utilisation pratique.

Pour atteindre ces buts, la présente invention propose un distributeur de jets d'air chargé de fragrance, comprenant une poche souple déformable de volume interne variable renfermant des microcapsules de fragrance, la poche étant initialement sensiblement vide d'air avec les microcapsules de fragrance intactes, les microcapsules de fragrance étant brisées lors d'une première entrée d'air dans la poche, de manière à charger l'air de la poche avec de la fragrance, la poche comprenant un orifice à travers lequel l'air pénètre dans la poche et sort de la poche en étant chargé avec de la fragrance, lorsque la poche varie de volume. Tout comme dans un vaporisateur à pompe, le distributeur de la présente invention produit un jet, qui se différencie cependant du jet du vaporisateur en ce qu'il s'agit d'un jet d'air, et non pas un jet de fines gouttelettes. L'air du jet du distributeur de la présente invention est chargé de fragrance sous forme de vapeur, et non pas sous forme liquide. Le distributeur de la présente invention constitue ainsi une sorte de distributeur hybride entre le vaporisateur à pompe et la bande à humer. Avant la première utilisation du distributeur, la poche ne contient que les microcapsules de fragrance intactes : ce n'est qu'à la première utilisation que la poche est gonflée en faisant rentrer pour la première fois de l'air extérieur à l'intérieur de la poche. Le déploiement de la poche a pour effet de briser les microcapsules de fragrance, et la fragrance peut ainsi charger l'air contenu à l'intérieur de la poche. En appuyant alors sur la poche, l'air chargé de fragrance est refoulé hors de la poche à travers l'orifice de distribution sous la forme d'un jet d'air chargé de fragrance.

Selon une forme de réalisation pratique, la poche peut comprendre deux parois dont une forme l'orifice, au moins une des parois étant au moins partiellement revêtue avec une couche comprenant des microcapsules de fragrance, les parois étant déplaçables l'une par rapport à l'autre à partir d'un état initial dans lequel elles sont en contact, les microcapsules intactes étant sensiblement hors de contact de l'air.

Avantageusement, les deux parois sont reliées ensemble sur leurs périphéries. De préférence, au moins une des parois comprend une feuille souple. La poche peut très simplement être réalisé à partir d'une feuille souple déformable qui est repliée sur elle-même puis soudée ensemble sur leurs périphéries. La couche de microcapsules de fragrance peut être appliquée sur une ou les deux surfaces internes de la poche ainsi constituée.

Selon une caractéristique intéressante de la présente invention, le distributeur peut en outre comprendre des moyens d'armement pour décoller les deux parois l'une de l'autre de manière à briser les microcapsules de fragrance et à faire pénétrer de l'air dans la poche. Ces moyens d'armement permettent ainsi d'initialiser le distributeur en brisant les microcapsules et en gonflant la poche avec de l'air simultanément.

Avantageusement, le distributeur peut en outre comprendre des moyens d'armement pour décoller les deux parois l'une de l'autre de manière à briser les microcapsules de fragrance et à faire pénétrer de l'air dans la poche.

Selon un autre aspect intéressant de la présente invention, les moyens de ressort coopèrent avec des moyens d'armement aptes à amener les moyens de ressort en sollicitation de la poche à partir d'un état initial dans lequel les moyens de ressort ne sollicitent pas la poche. Ainsi, les moyens d'armement, en plus de briser les microcapsules et de faire pénétrer pour la première fois l'air dans la poche, permettent également d'activer les moyens de ressort de manière à agir sur la poche en la sollicitant vers son état de volume maximal. Ces moyens de ressort ne sont utilisables qu'après la mise en fonctionnement des moyens d'armement. Les moyens de ressort permettent une utilisation répétée du distributeur de manière à pouvoir distribuer plusieurs jets d'air chargé. Les moyens de ressort remplissent ainsi une fonction de rappel permettant de ramener la poche dans son état de volume maximal.

Selon une forme de réalisation pratique, les moyens de ressort peuvent comprendre une plaque avant et une plaque arrière, la poche étant située entre les plaques avant et arrière, la poche étant fixée aux plaques avant et arrière, les moyens d'armement comprenant des moyens d'entretoise sélectivement déplaçables entre une position inopérante dans laquelle les deux plaques s'étendent sensiblement parallèlement correspondant à un état de volume minimal de la poche et une position opérante dans laquelle les plaques sont éloignées au moins localement l'une de l'autre, la plaque avant étant déplaçable par rapport à la plaque arrière de manière à écraser la poche située entre elles.

Avantageusement, des moyens d'actionnement sont prévus pour positionner les moyens d'entretoise entre les deux plaques de manière à les éloigner, les moyens d'actionnement comprenant un organe de traction.

Avantageusement, les moyens d'entretoise et les moyens d'actionnement sont réalisés de manière monobloc.

Avantageusement, les moyens d'entretoise comprennent un rabat articulé déplaçable entre les positions inopérante et armée, ledit rabat et l'organe de traction étant réalisés de manière monobloc.

Avantageusement, l'organe de traction forme un étrier comprenant deux branches reliées ensemble par une âme commune.

Avantageusement, la poche est fixée à une plaque entre les deux branches de l'étrier.

Avantageusement, les moyens de ressort sont formés par au moins un parmi la plaque avant, la plaque arrière et les moyens d'entretoise.

Avantageusement, l'orifice débouche latéralement à travers une des plaques.

L'esprit de la présente invention est de réaliser un jet ou flux de fragrance sous forme de vapeur, et non pas sous la forme de fines gouttelettes de liquide divisées. L'utilisateur peut directement humer le jet d'air chargé de fragrance en le dirigeant en direction de son nez, ce qui n'est pas possible avec un jet de fines gouttelettes liquide divisées. L'inventivité de la présente invention repose principalement sur le fait que l'on se sert de la première entrée d'air dans la poche pour décoller les parois de la poche et ainsi briser les microcapsules de fragrance. Ainsi, l'utilisateur n'a même pas conscience qu'il brise des microcapsules de fragrance lorsqu'il actionne le distributeur pour faire pénétrer de l'air à l'intérieur de la poche.

L'avantage des microcapsules de fragrance, par rapport à une matière poreuse imbibée de fragrance liquide, est qu'il n'y a aucun risque de fuite à travers l'orifice du distributeur, qui peut rester ouvert. En d'autres termes, il n'y a même pas besoin de prévoir un organe d'obturation pour l'orifice du distributeur, lorsque l'on utilise des microcapsules de fragrance. Toutefois, il n'est pas exclu, dans le cas de la présente invention, de remplacer les microcapsules de fragrance par un matériau poreux imbibé de fragrance liquide.

L'invention sera maintenant plus amplement décrite en référence aux dessins joints donnant à titre d'exemple non limitatif un mode de réalisation d'un distributeur selon l'invention.

Sur les figures :
la figure 1 est une vue de dessus en perspective d'un distributeur selon l'invention dans un état non fini pour montrer sa structure interne, et
les figures 2, 3 et 4 sont des vues en coupe transversale verticale à travers le distributeur de la figure 1.

Le distributeur de jets d'air chargé de fragrance de la présente invention comprend une poche souple déformable 3 qui est avantageusement associée à des moyens de ressort et d'armement qui vont servir à initialiser la poche et à faire varier son volume interne, comme on le verra ci-après. Les moyens de ressort et d'armement enveloppent la poche à la manière d'un étui.

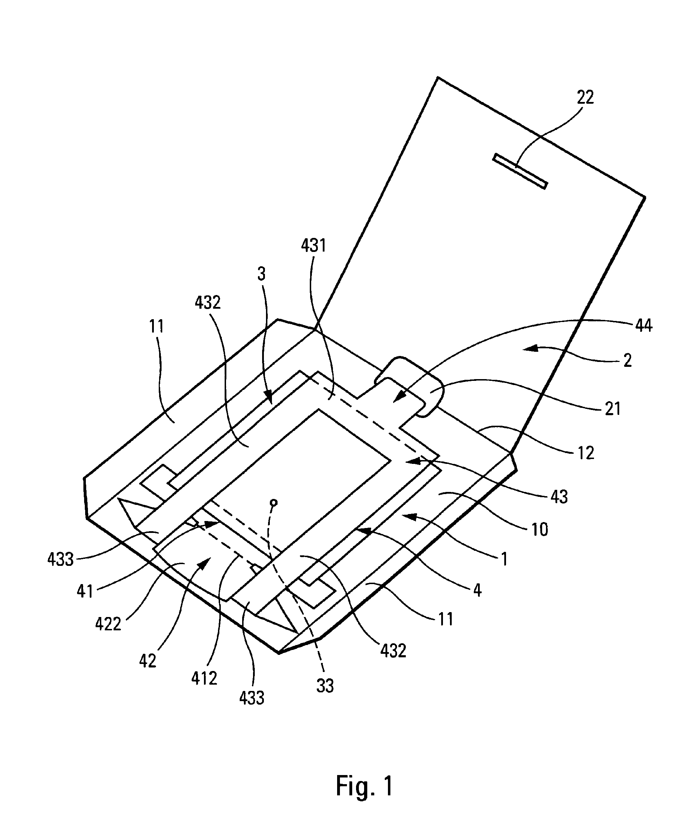

La poche souple déformable 3 comprend deux parois 31 dont une forme un orifice de distribution 33. Sur la figure 1, la queue d'attache de l'orifice 33 était représentée en pointillée pour signifier que l'orifice est situé sur la face inférieure de la poche, qui n'est pas visible sur la figure 1. Les deux parois 31 de la poche peuvent par exemple être réalisées à partir d'une feuille souple repliée sur elle-même et soudée sur la périphérie. Il est également possible de réaliser la poche à partir de deux feuilles souples séparées. Il est également possible de réaliser la poche avec une feuille souple et une paroi rigide ou semi-rigide. Il est également envisageable d'utiliser deux parois rigides reliées ensemble par un élément souple, par exemple sous la forme d'un soufflet. Lorsque la poche utilise une ou plusieurs feuilles souples, celles-ci peuvent par exemple être réalisées à partir d'un laminé d'aluminium et de matière plastique. Ces feuilles peuvent être soudées ensembles par un simple thermosoudage de la matière plastique.

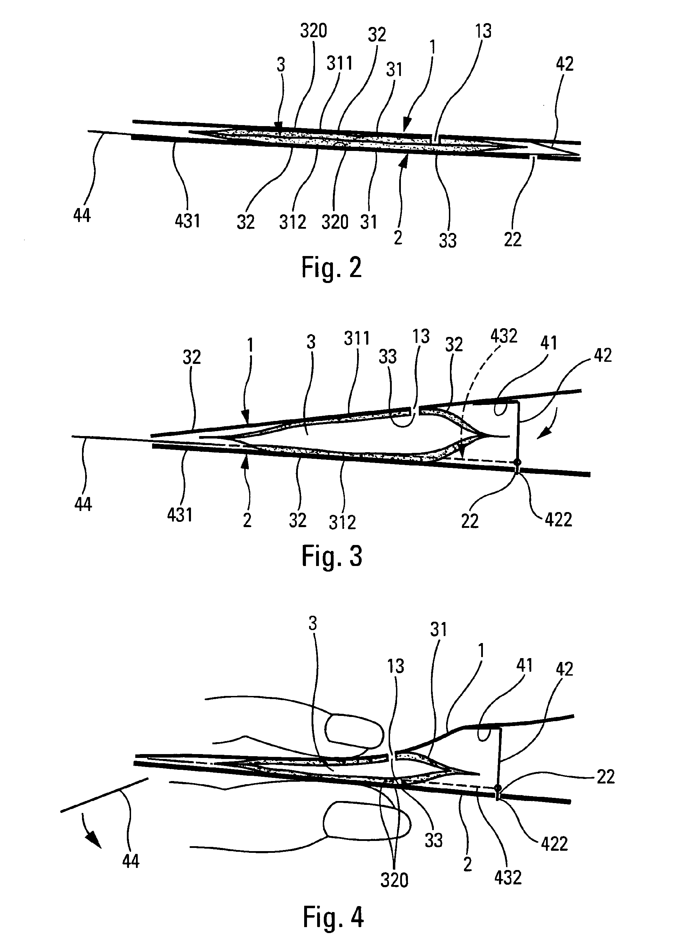

Selon l'invention, la poche souple déformable 3 contient des microcapsules de fragrance 320, par exemple sous la forme d'une ou de deux couches 32 de microcapsules de fragrance 320 appliquées sur une ou les deux parois 31 de la poche. Sur les figures 2 à 4, on peut observer que la poche contient deux couches adjacentes 32 de microcapsules de parfum 320, respectivement appliquées sur chacune des parois 31 de la poche. L'épaisseur des couches 32 a été volontairement exagéré pour pouvoir les discerner sur les figures. En réalité, les couches sont extrêmement minces et imperceptibles sur des vues en coupe. De manière similaire, les microcapsules 320 ont été représentées dans les couches 32, mais ceci ne reflète pas la réalité. A l'état initial, comme représenté sur les figures 1 et 2, les deux couches 32 de microcapsules de fragrance 320 sont adjacentes et collées l'une à l'autre. Dans cet état initial, la poche ne contient que peu ou pas d'air du tout. Toutefois, l'orifice de distribution 33 peut rester ouvert, étant donné qu'il n'y a aucun risque de fuite du fait de l'utilisation de microcapsules de fragrance. D'autre part, il n'y a également aucun risque que de l'air pénètre à l'intérieur de la poche, étant donné que les deux couches 32 sont collées l'une à l'autre. Il en aurait été de même dans le cas où la poche 3 ne contiendrait qu'une seule couche 32 de microcapsules de fragrance : la couche 32 serait alors disposée entre les deux parois 31 de la poche. La poche présente alors une épaisseur de l'ordre du millimètre.

A la place des microcapsules de fragrance, on peut également utiliser dans le cadre de l'invention une matière poreuse imbibée de fragrance. Toutefois, la matière poreuse imbibée présente le désavantage de pouvoir fuir lorsqu'elle est soumise à une contrainte, comme c'est le cas lorsque le distributeur est disposé entre les pages d'un magazine. Dans les autres cas d'utilisation où le distributeur n'est pas soumis à une contrainte, le remplacement des microcapsules par une matière poreuse imbibée est envisageable.

Les microcapsules de fragrance 320, afin de pouvoir libérer leur contenu, doivent être brisées. Il est bien connu dans l'art antérieur de briser ces microcapsules en les détachant ou en les arrachant d'une feuille, comme c'est la cas avec les bande à humer. Dans le cas de la poche souple et déformable 3 de l'invention, il est donc nécessaire de décoller ou de détacher les deux couches 32 l'une de l'autre. Cette opération pourrait être réalisée manuellement en tirant directement sur les parois 31 de la poche. On pourrait par exemple prévoir une languette de préhension sur chacune des parois 31 de sorte que l'utilisateur pourrait tirer sur les parois 31 en tenant les languettes. La présente invention propose une autre technique pour décoller les couches l'une de l'autre et produire le jet d'air chargé de fragrance. Pour cela, la présente invention prévoit des moyens d'armement et de ressort qui permettent dans un premier temps de décoller les couches 32 l'une de l'autre et ensuite d'actionner la poche pour émettre les jets d'air chargés de fragrance.

Les moyens de ressort et d'armement se présentent ici sous la forme d'une plaque supérieure 2 et d'une plaque inférieure 1, associées à un rabat pivotant 42 et un organe de traction 43, 44 pour faire pivoter le rabat. Le rabat 42 remplit une fonction de moyens d'armement permettant d'activer les moyens de ressort. Il peut également assurer une fonction de ressort. Au moins une des deux plaques est élastiquement déformable, de manière à remplir une fonction de ressort, si le rabat ne la remplit pas déjà. De préférence, les deux plaques sont élastiquement déformables et le rabat est rigide. Les deux plaques 1 et 2 peuvent être réalisées à partir d'un morceau de plaque unique relié par un pli de liaison 12, comme c'est le cas sur les figures. Une découpe ou échancrure 21 est formée au niveau du pli de liaison 12. L'échancrure peut être réalisée par une fenêtre qui s'étend à cheval sur le pli et qui est ensuite repliée sur elle-même.

La plaque 1 est formée par un pan 10 et deux volets latéraux 11 qui sont repliables sur le pan. La plaque 1 est pourvue d'un trou 13. La plaque 2 est reliée au pan 10 par le pli 12. La plaque 2 a sensiblement la même taille que le pan 10, de sorte qu'ils peuvent se superposer.

Un talon de fixation 41 est avantageusement collé sur le pan 10. Le talon peut aussi être réalisé d'une seule pièce avec la plaque 1. Le rabat 42 être articulé par rapport au talon 41 par pivotement autour d'une ligne 412. Le rabat va servir de moyens d'armement sous la forme de moyens d'entretoise, comme on le verra ci-après. Le rabat est en outre pourvu d'une petite languette de blocage 422 dont la fonction sera expliquée ci-après. Le talon et le rabat peuvent être réalisés d'une seule pièce. Le rabat est avantageusement situé à proximité du bord opposé au pli 12.

Quant à la plaque 2, elle est formée avec une fente 22 destinée à recevoir la languette de blocage 422 de la plaque 1.

Les plaques sont destinées à être fixées, avantageusement thermosoudées, ensemble au niveau des volets 11. On obtient ainsi une sorte d'enveloppe scellée sur trois côtés et ouvert sur le côté où est formé le rabat articulé 42. La poche déformable 3 est disposée entre les deux plaques à l'intérieur de l'enveloppe qu'elles forment ensemble. L'orifice de distribution 33 est positionné avantageusement au niveau du trou 13. La poche 3 peut avantageusement être fixée à l'intérieur de l'enveloppe constituée par les deux plaques. La poche peut par exemple être fixée en deux zones 311 et 312 à chacune des plaques.

Lorsque le rabat 42 s'étend dans le même plan que le restant de la plaque 1, comme représenté sur les figures 1 et 2, la plaque 2 s'étend sensiblement parallèlement à la plaque 1, avec la poche déformable 3 intercalée entre elles. La poche déformable 3 ne contient avantageusement que la ou les couche(s) 32 de microcapsules de fragrance encore intactes, de sorte qu'elle présente une configuration particulièrement plate. Ainsi, lorsque disposée entre les deux plaques 1 et 2, elle ne crée qu'une faible surépaisseur, et ainsi les deux plaques semblent être superposées avec presque rien entre elles. Ceci est représenté sur la figure 2, où l'épaisseur des deux couches 32 de microcapsules de fragrance a été exagérée pour pouvoir les discerner. L'épaisseur cumulée réelle du distributeur peut être de l'ordre de 2 à 3 mm. Le distributeur est alors dans un état de repos approprié pour son transport et son stockage. La poche déformable 3, qui forme les parois d'actionnement, n'est soumise à aucune contrainte. De même, les plaques 1 et 2 ne sont soumises à aucune contrainte.

Pour armer le distributeur, il suffit de faire pivoter le rabat 42 autour de la ligne d'articulation 412 en direction de la plaque 2, comme cela est représenté par la petite flèche sur la figure 3. La ligne de pliage 412 est avantageusement réalisée de manière courbe, par exemple en forme d'arc de cercle. De manière symétrique, le bord du rabat où est formée la languette peut également être réalisé de manière courbe. Ainsi, lorsque le rabat 42 pivote en direction de la plaque 2, les plaques prennent une courbure correspondant à la ligne de pliage et au bord libre. Le rabat est articulé par pivotement jusqu'à ce que son bord libre vienne en appui sur la plaque 2 et sa petite languette de blocage 422 vienne se loger à l'intérieur de la fente 22 formée dans la plaque 2. Ceci est représenté sur la figure 3. Le rabat s'étend alors de manière sensiblement perpendiculaire, à la fois à la plaque 1 et à la plaque 2. Du fait de l'engagement de la languette 422 dans la fente 22, le rabat est bloqué en position. Le rabat peut par exemple être réalisé de manière rigide. Il constitue ainsi des moyens d'entretoise qui permettent de maintenir les deux plaques éloignées l'une de l'autre.

Etant donné que la poche 3 est fixée à la plaque 1 en 311 et la plaque 2 en 312, l'écartement des deux plaques du fait de l'interposition du rabat d'entretoise a pour effet également d'écarter les parois de la poche. Les deux couches 32 de microcapsules de fragrance sont ainsi détachées ou arrachées l'une de l'autre, ce qui a pour effet de briser les microcapsules de fragrance qui libèrent alors leur contenu de fragrance. L'augmentation du volume interne de la poche s'accompagne par une première entrée d'air à travers l'orifice de distribution 33. L'écartement des plaques n'est possible que si de l'air peut pénétrer à l'intérieur du réservoir. Selon l'invention, les plaques sont réalisées en un matériau élastiquement déformable qui leur confère cette caractéristique d'élasticité de rappel. Ainsi, une fois le distributeur dans la configuration représentée sur la figure 3, on peut l'actionner en appuyant sur la plaque 1 à l'aide d'un doigt et en maintenant l'autre plaque à l'aide d'un autre doigt. Ceci est représenté sur la figure 4. Les plaques peuvent chacune former des moyens de ressort de rappel. Dans le mode de réalisation des figures, seule la plaque 1 forme des moyens de rappel élastique, alors que la plaque 2 peut être parfaitement rigide et servir de surface de réaction. On peut également imaginer que seul le rabat est élastiquement déformable et les plaques rigides. Un rabat souple avec une plaque déformable est aussi envisageable.

On peut rapprocher les plaques l'une de l'autre en déformant la poche. Ceci a pour effet de mettre l'air de la poche 3 sous pression et ainsi de refouler de l'air chargé de fragrance sous la forme d'un jet.

On peut ainsi remarquer que le rabat d'entretoise 42 forme des moyens d'armement permettant d'amener les plaques dans une configuration espacée.

Les moyens de ressort et d'armement se présentent ici sous la forme d'une enveloppe qui entoure la poche 3. On peut également imaginer des formes de réalisation dans lesquelles les moyens de ressort et d'armement se présentent sous la forme de deux bandes ou lames étroites dont une comprend un rabat d'entretoise d'armement. La poche souple 3 serait alors disposée entre ces deux bandes.

Selon l'invention, le distributeur de produit fluide est pourvu de moyens d'actionnement qui sont désignés dans leur ensemble par la référence numérique 4 sur la figure 1. Ces moyens d'actionnement peuvent inclure dans leur définition le rabat 42 ainsi que le talon de fixation 41, étant donné qu'ils peuvent être réalisés de manière monobloc avec le restant des moyens d'actionnement 4. Les moyens d'actionnement 4 comprennent un organe de traction 43, 44 qui s'étend entre le rabat 42 et l'échancrure 21. L'organe de traction est relié, avantageusement de manière monobloc au bord du rabat 42 où est formé la languette de blocage 422. D'autre part, l'organe de traction comprend une patte de traction 44 initialement positionnée dans l'échancrure 21. En tirant sur la patte de traction 44, l'organe de traction transmet la force de traction au rabat 42 qui alors sollicité en pivotement depuis sa position inopérante représentée sur la figure 2 jusqu'à sa position armée représentée sur la figure 3. La position finale est celle où la languette de blocage 422 vient se loger dans la fente 22 de la plaque 2. L'utilisateur va immédiatement comprendre qu'il faut saisir la patte de traction 44 et tirer dessus. L'organe de traction s'étend entre les deux plaques 1 et 2.

Il s'agit là du concept général réalisé pour les moyens d'actionnement de l'invention.

Les figures destinées à illustrer la présente invention donne cependant un mode de réalisation très pratique de ces moyens d'actionnement 4. Plus particulièrement en référence à la figure 1, on peut voir que les moyens d'actionnement 4 forment l'organe de traction qui comprend un étrier de transmission de force 43 ainsi qu'une patte de traction 44. L'étrier et la patte peuvent être réalisés de manière monobloc, ou en variante, la patte 44 peut être fixée, par exemple par collage, sur l'étrier 43. L'étrier 43 comprend deux branches latérales sensiblement parallèles 432 reliées au niveau d'une de leurs extrémités à une âme commune 431. Les branches 432 s'étendent sensiblement parallèlement aux volets 11 de la plaque 1. L'âme 431 est située à proximité de l'échancrure 21. Les branches 432 définissent des extrémités de liaison 433 reliées au rabat 42 de part et d'autre de la languette de blocage 422. L'étrier 43 et le rabat 42 peuvent être réalisés de manière monobloc, avantageusement avec le talon de fixation 41. La patte de traction 44 est reliée à l'étrier 43 au niveau de l'âme commune 431. Initialement, avant la première utilisation, le rabat 42 est plaqué sur la plaque 1. La patte de traction 44 est alors positionnée dans l'échancrure 21 sans en faire saillie à l'extérieur. L'utilisateur peut se saisir de la patte de traction 44 et tirer dessus de manière à faire pivoter le rabat 42. En continuant à tirer, l'utilisateur va détacher la patte 44 de l'étrier 43. Ceci est visible sur la figure 4.

La poche déformable 3 est disposée entre l'étrier 43 et la plaque 1, comme on peut le voir sur la figure 1, la poche 3 étant représentée en trait pointillé. La forme en étrier permet d'une part une meilleure répartition des forces de traction sur le rabat 42, mais permet également une fixation centrée de la poche 3 au niveau de la zone 312, représenté sur les figures 2 et 3, qui est situé entre les deux branches. La forme en étrier permet également de réaliser un contact de butée entre l'âme commune 431 et le pli 12 en fin de traction, ce qui a pour conséquence de détacher la patte 44 de l'étrier 43.

## Revendications

1. Distributeur de jets d'air chargé de fragrance, **caractérisé en ce qu'**il comprend une poche souple déformable (3) de volume interne variable renfermant des microcapsules de fragrance (320), la poche (3) étant initialement sensiblement vide d'air avec les microcapsules de fragrance (320) intactes, les microcapsules de fragrance (320) étant brisées lors d'une première entrée d'air dans la poche (3) de manière à charger l'air de la poche (3) avec de la fragrance, la poche comprenant un orifice (33) à travers lequel l'air pénètre dans la poche (3) et sort de la poche (3) en étant chargé avec de la fragrance, lorsque la poche varie de volume.

2. Distributeur selon la revendication 1, dans lequel la poche (3) comprend deux parois (31) dont une forme l'orifice (33), au moins une des parois (31) étant au moins partiellement revêtue avec une couche (32) comprenant des microcapsules de fragrance (320), les parois (31) étant déplaçables l'une par rapport à l'autre à partir d'un état initial dans lequel elles sont en contact, les microcapsules intactes étant sensiblement hors de contact de l'air.

3. Distributeur selon la revendication 1 ou 2, dans lequel les deux parois (31) sont reliées ensemble sur leurs périphéries.

4. Distributeur selon la revendication 1, 2 ou 3, dans lequel au moins une des parois comprend une feuille souple (31).

5. Distributeur selon l'une quelconque des revendications précédentes, comprenant en outre des moyens d'armement (42) pour décoller les deux parois (31) l'une de l'autre de manière à briser les microcapsules de fragrance (320) et à faire pénétrer pour la première fois de l'air dans la poche (3).

6. Distributeur selon la revendication 5, comprenant en outre des moyens de ressort (1, 2, 42) pour solliciter la poche (3) dans un état de volume maximal, la poche étant écrasable contre l'action des moyens de ressort pour expulser l'air chargé de fragrance hors de la poche.

7. Distributeur selon la revendication 6, dans lequel les moyens de ressort (1, 2, 42) coopèrent avec les moyens d'armement (42) pour amener les moyens de ressort en sollicitation de la poche (3) à partir d'un état initial dans lequel les moyens de ressort ne sollicitent pas la poche.

8. Distributeur selon la revendication 7, dans lequel les moyens de ressort comprennent une plaque avant (1) et une plaque arrière (2), la poche (30) étant située entre les plaques avant et arrière, la poche étant fixée aux plaques avant et arrière, les moyens d'armement comprenant des moyens d'entretoise (42) sélectivement déplaçables entre une position inopérante dans laquelle les deux plaques s'étendent sensiblement parallèlement correspondant à un état de volume minimal de la poche et une position opérante dans laquelle les plaques sont éloignées au moins localement l'une de l'autre, la plaque avant étant déplaçable par rapport à la plaque arrière de manière à écraser la poche située entre elles.

9. Distributeur selon la revendication 8, dans lequel des moyens d'actionnement (4) sont prévus pour positionner les moyens d'entretoise entre les deux plaques de manière à les éloigner, les moyens d'actionnement comprenant un organe de traction (43, 44).

10. Distributeur selon la revendication 9, dans lequel les moyens d'entretoise (42) et les moyens d'actionnement (4) sont réalisés de manière monobloc.

11. Distributeur selon la revendication 9, dans lequel les moyens d'entretoise comprennent un rabat articulé (42) déplaçable entre les positions inopérante et armée, ledit rabat et l'organe de traction (43) étant réalisés de manière monobloc.

12. Distributeur selon la revendication 9 ou 11, dans lequel l'organe de traction forme un étrier (43) comprenant deux branches (432) reliées ensemble par une âme commune (431).

13. Distributeur selon la revendication 12, dans lequel la poche (3) est fixée à une plaque (1) entre les deux branches de l'étrier.

14. Distributeur selon l'une quelconque des revendications 6 à 13, dans lequel les moyens de ressort sont formés par au moins un parmi la plaque avant (1), la plaque arrière (2) et les moyens d'entretoise (42).

15. Distributeur selon la revendication 7, dans lequel l'orifice (33) débouche latéralement à travers une des plaques (1, 2).

## Patentansprüche

1. Spender für einen mit Duft beladenen Luftstrahl, **dadurch gekennzeichnet, dass** er eine flexible, verformbare Tasche (3) mit einem veränderlichen Innenvolumen aufweist, in dem Mikroduftkapseln (320) enthalten sind, wobei die Tasche (3) zunächst im Wesentlichen luftleer ist und die Mikroduftkapseln (320) unbeschädigt sind, wobei die Mikroduftkapseln (320) bei einem ersten Lufteintritt in die Tasche (3) so zerreißen, dass die Luft der Tasche (3) mit dem Duft beladen wird, wobei die Tasche eine Öffnung (33) aufweist, durch die die Luft in die Tasche (3) eintritt und aus der Tasche (3) austritt, wobei sie mit dem Duft beladen ist, wenn sich das Volumen der Tasche ändert.

2. Spender nach Anspruch 1, wobei die Tasche (3) zwei Wände (31) aufweist, von denen eine die Öffnung (33) bildet, wobei mindestens eine der Wände (31) zumindest teilweise mit einer Schicht (32) ausgekleidet ist, die Mikroduftkapseln (320) umfasst, wobei die Wände (31) von einem ursprünglichen Zustand, in dem sie in Kontakt stehen, in Bezug zueinander verschiebbar sind, wobei die unbeschädigten Mikrokapseln im Wesentlichen außer Kontakt mit der Luft sind.

3. Spender nach Anspruch 1 oder 2, wobei die beiden Wände (31) über ihren Umfang miteinander verbunden sind.

4. Spender nach Anspruch 1, 2 oder 3, wobei mindestens eine der Wände eine flexible Folie (31) aufweist.

5. Spender nach einem der vorhergehenden Ansprüche, des Weiteren aufweisend Spannmittel (42), um die beiden Wände (31) voneinander zu lösen, so dass die Mikroduftkapseln (320) zerplatzen und dass das erste Mal Luft in die Tasche (3) eindringt.

6. Spender nach Anspruch 5, des Weiteren aufweisend Federmittel (1, 2, 42), um die Tasche (3) in einem Zustand maximalen Volumens zu belasten, wobei die Tasche gegen die Wirkung der Federmittel zusammengedrückt werden kann, um die mit Duft beladene Luft aus der Tasche auszutreiben.

7. Spender nach Anspruch 6, wobei die Federmittel (1, 2, 42) mit den Spannmitteln (42) zusammenwirken, um die Federmittel aus einem ursprünglichen Zustand, in dem die Federmittel die Tasche nicht belasten, zu einer Belastung der Tasche (3) zu bringen.

8. Spender nach Anspruch 7, wobei die Federmittel eine Vorderplatte (1) und eine Rückplatte (2) aufweisen, wobei sich die Tasche (30) zwischen der Vorder- und der Rückplatte befindet, wobei die Tasche an der Vorder- und der Rückplatte befestigt ist, wobei die Spannmittel Verstrebungsmittel (42) aufweisen, die wahlweise zwischen einer inaktiven Position, in der sich die beiden Platten in etwa parallel zueinander erstrecken, was einem Zustand minimalen Volumens der Tasche entspricht, und einer aktiven Position verschiebbar sind, in der die Platten zumindest lokal voneinander entfernt sind, wobei die Vorderplatte in Bezug auf die Rückplatte so verschiebbar ist, dass sie die zwischen diesen befindliche Tasche zusammendrückt.

9. Spender nach Anspruch 8, wobei Betätigungsmittel (4) vorgesehen sind, um die Verstrebungsmittel zwischen den beiden Platten so anzuordnen, dass diese voneinander entfernt sind, wobei die Betätigungsmittel ein Zugelement (43, 44) aufweisen.

10. Spender nach Anspruch 9, wobei die Verstrebungsmittel (42) und die Betätigungsmittel (4) in einem Stück gefertigt sind.

11. Spender nach Anspruch 9, wobei die Verstrebungsmittel eine angelenkte Klappe (42) aufweisen, die zwischen der inaktiven und der gespannten Position verschiebbar ist, wobei die Klappe und das Zugelement (43) aus einem Stück gefertigt sind.

12. Spender nach Anspruch 9 oder 11, wobei das Zugelement einen Bügel (43) bildet, der zwei Schenkel (432) aufweist, die durch einen gemeinsamen Steg (431) miteinander verbunden sind.

13. Spender nach Anspruch 12, wobei die Tasche (3) an einer Platte (1) zwischen den beiden Bügelschenkeln befestigt ist.

14. Spender nach einem der Ansprüche 6 bis 13, wobei die Federmittel mindestens entweder durch die Vorderplatte (1), die Rückplatte (2) oder die Verstrebungsmittel (42) gebildet sind.

15. Spender nach Anspruch 7, wobei die Öffnung (33) seitlich durch eine der Platten (1, 2) durchgeht.

## Claims

1. A dispenser for dispensing jets of air laden with fragrance, the dispenser being **characterized in that** it includes a deformable flexible pouch (3) of variable internal volume containing fragrance microcapsules (320), the pouch (3) initially being substantially empty of air with the fragrance microcapsules (320) intact, the fragrance microcapsules (320) being broken the first time air enters into the pouch (3), in such a manner as to load the air in the pouch (3) with fragrance, the pouch including an orifice (33) through which the air penetrates into the pouch (3), and through which the fragrance-laden air leaves the pouch (3) when the volume of the pouch is varied.

2. A dispenser according to claim 1, in which the pouch (3) includes two walls (31) with the orifice (33) formed in one of them, at least one of the walls (31) being covered at least in part with a layer (32) comprising fragrance microcapsules (320), the walls (31) being movable relative to each other from an initial state in which they are in contact, the intact microcapsules being substantially not in contact with the air.

3. A dispenser according to claim 1 or claim 2, in which the two walls (31) are connected together via their peripheries.

4. A dispenser according to claim 1, or 2, or 3, in which at least one of the walls comprises a flexible sheet (31).

5. A dispenser according to any preceding claim, further including primer means (42) for separating the two walls (31) from each other in such a manner as to break the fragrance microcapsules (320), and to cause air to penetrate into the pouch (3) for the first time.

6. A dispenser according to claim 5, further including spring means (1, 2, 42) for urging the pouch (3) into a maximum-volume state, the pouch being flattenable against the action of the spring means so as to expel the fragrance-laden air out from the pouch.

7. A dispenser according to claim 6, in which the spring means (1, 2, 42) co-operate with the primer means (42) for causing the spring means to act on the pouch starting from an initial state in which the spring means do not act on the pouch.

8. A dispenser according to claim 7, in which the spring means comprise a front plate (1) and a rear plate (2), the pouch (30) being situated between the front and rear plates, the pouch being fastened to the front and rear plates, the primer means comprising spacer means (42) that are selectively moved between an inoperative position in which the two plates extend substantially parallel, corresponding to a minimum-volume state of the pouch, and an operating position in which the plates are spaced apart from each other, at least locally, the front plate being movable relative to the rear plate in such a manner as to flatten the pouch situated between them.

9. A dispenser according to claim 8, in which actuator means (4) are provided for positioning the spacer means between the two plates in such a manner as to move them apart, the actuator means comprising a traction member (43, 44).

10. A dispenser according to claim 9, in which the spacer means (42) and the actuator means (4) are made as a single unit.

11. A dispenser according to claim 9, in which the spacer means comprise a hinged flap (42) that is movable between the inoperative position and the primed position, said flap and the traction member (43) being made as a single unit.

12. A dispenser according to claim 9 or claim 11, in which the traction member forms a fork (43) comprising two branches (432) that are interconnected via a common web (431).

13. A dispenser according to claim 12, in which the pouch (3) is fastened to a plate (1) between the two branches of the fork.

14. A dispenser according to any one of claims 6 to 13, in which the spring means are formed by at least one of: the front plate (1); the rear plate (2); and the spacer means (42).

15. A dispenser according to claim 7, in which the orifice (33) opens out laterally through one of the plates (1, 2).
